# EUROPÄISCHE PATENTSCHRIFT

(11) **EP 0 554 844 B1**
(45) Veröffentlichungstag und Bekanntmachung des Hinweises auf die Patenterteilung: **26.06.1996**
(21) Anmeldenummer: 93101625.7
(22) Anmeldetag: 03.02.1993
(51) Int. Cl.: C07C 269/00, C07C 271/22

(54) **Verfahren zur Herstellung von Omega-(O-substituierten Urethano)alkylcarbonsäureestern**
Process for the preparation of esters of omega (O-substituted urethane)alkylcarboxylic acids
Procédé pour la préparation d'esters d'oméga (O-substitué uréthano)alkcoylcarboxyliques

(30) Priorität: 07.02.1992 DE 4203457
(43) Veröffentlichungstag der Anmeldung: 11.08.1993
(73) Patentinhaber: BASF Aktiengesellschaft, D-67063 Ludwigshafen (DE)
(72) Erfinder: Merger, Franz, Dr., W-6710 Frankenthal (DE); Brudermüller, Martin, Dr., W-6800 Mannheim 1 (DE)

(56) Entgegenhaltungen:
- JOURNAL OF ORGANIC CHEMISTRY. Bd. 48, Nr. 14, 15. Juli 1983, EASTON US Seiten 2424 - 6 P.A. GRIECO ET. AL. 'A mild two-step method for the hydrolysis/methanolysis of secondary amides and lactams'
- AUSTRALIAN JOURNAL OF CHEMISTRY Bd. 29, Nr. 10, Oktober 1976, EAST MELBOURNE AU Seiten 2651 - 65 T. DUONG ET. AL. 'Central nervous sysrem active compounds. I. The synthesis of some caprolactam derivatives substituted at N1, C2 and C3' 3 Seite 2654, Schema 3 *

## Beschreibung

Gegenstand der Erfindung ist ein Verfahren zur Herstellung von ω-(O-substituierten Urethano)alkylcarbonsäureestern aus Lactamen.

Aus der US-Patentschrift 3 078 301 ist bekannt, daß man ω-(O-Alkylurethano)carbonsäurealkylester erhält, wenn man zunächst ein Lactam mit einem Alkalihydroxid zum entsprechenden Alkalilactam umsetzt und in einer zweiten Stufe Chlorkohlensäurealkylester mit Alkalilactam umsetzt. Dieses Verfahren hat den Nachteil, daß es zweistufig verläuft und zudem Chloridionen enthaltende behandlungsbedürftige Abwässer anfallen.

Nach einem anderen in J. Org. Chem. Band 48, Seiten 2424 bis 2426 (1983) beschriebenen Verfahren wird δ-Valerolactam mit Oxydiameisensäure-tert.-butylester zunächst in N-tert.-Butoxycarbonylvalerolactam übergeführt, das anschließend in einer zweiten Stufe mit 1,1 Äquivalenten Natriummethylat zu 5-(O-tert.-Butylurethano)valeriansäuremethylester gespalten wird.

Schließlich ist aus Austr. J. of Chem. Band 29, Seiten 2651 bis 2665 (1976) bekannt, daß man Caprolactam zunächst in das Natriumsalz überführt und dieses Natriumsalz des Caprolactams mit Diethylcarbonat umsetzt und 6-(O-Ethylurethano)carbonsäureethylester erhält. Es wird ausdrücklich darauf hingewiesen, daß vor der Umsetzung das gesamte Caprolactam mit Natriumhydrid in das Natriumsalz übergeführt werden muß. Beide letztgenannten Verfahren haben den Nachteil, daß sie zweistufig verlaufen und bei der Herstellung von Alkalilactamen Lösungsmittel mitverwendet werden müssen.

Es war deshalb die technische Aufgabe gestellt, ein Verfahren zur Herstellung von ω-(O-substituierten Urethano)carbonsäureestern zur Verfügung zu stellen, bei dem man die Umsetzung in einer Stufe durchführt und die Umsetzung mit guten Ausbeuten verläuft, keine Chlor enthaltende Abwässer anfallen und keine zusätzlichen Lösungsmittel mitverwendet werden müssen.

Diese Aufgabe wird gelöst in einem Verfahren zur Herstellung von ω-(O-substituierten Urethano)alkylcarbonsäureestern, dadurch gekennzeichnet, daß man ein Lactam mit 4 bis 9 Ringgliedern, das Alkyl-, Alkenyl-, Cycloalkyl- oder Aralkylgruppen mit bis zu 12 Kohlenstoffatomen als Substituenten haben kann, mit Kohlensäurediestern von Alkanolen, Alkenolen, Cycloalkanolen oder Aralkanolen mit bis zu 16 Kohlenstoffatomen bei einer Temperatur von 25 bis 300°C in Gegenwart von katalytisch wirksamen Mengen einer Base umsetzt.

Das neue Verfahren hat den Vorteil, daß es mit hohen Ausbeuten in einer Stufe verläuft, keine behandlungsbedürftigen chloridhaltigen Abwässer anfallen und keine zusätzlichen Lösungsmittel mitverwendet werden müssen.

Erfindungsgemäß geht man von Lactamen mit 4 bis 9 Ringgliedern aus, die Alkyl-, Alkenyl, Cycloalkyl- oder Aralkylgruppen mit bis zu 12 Kohlenstoffatomen als Substituenten haben können. Vorteilhaft haben die verwendeten Lactame bis zu 2 Substituenten insbesondere Alkylreste mit 1 bis 4 Kohlenstoffatomen. Ganz besonders bevorzugt sind Lactame mit 5 bis 9 Ringgliedern, die keine weiteren Substituenten haben. Geeignete Lactame sind beispielsweise Azetidinon, Butyrolactam(2-Pyrrolidinon), δ-Valerolactam(2-Piperidinon), ε-Caprolactam, 7-Aminoheptansäurelactam und ω-Caprylactam.

Die vorgenannten Lactame werden mit Kohlensäurediestern von Alkenolen, Alkanolen, Cycloalkanolen oder Aralkanolen mit bis zu 16 Kohlenstoffatomen vorteilhaft im Überschuß umgesetzt. Die in den Kohlensäurediestern enthaltenen Alkoholteile können gleich oder verschieden sein. Die Alkanole, Alkenole, Cycloalkanole oder Aralkanole können auch Alkoxyreste mit 1 bis 4 Kohlenstoffatomen haben. Bevorzugt sind Kohlensäurediester, die sich von Alkanolen oder Alkenolen mit 1 bis 6 Kohlenstoffatomen, Cyclohexanol oder Benzylalkohol ableiten. Besondere Bedeutung haben Kohlensäurediester von Alkanolen oder Alkenolen mit 1 bis 3 Kohlenstoffatomen erlangt. Geeignete Kohlensäurediester sind beispielsweise Dimethylcarbonat, Diethylcarbonat, Dipropyl-, Diisopropylcarbonat, Diallylcarbonat, Dimethallylcarbonat, Di-n-Butylcarbonat, Dicyclohexylcarbonat oder Diisobutylcarbonat.

Die Umsetzung wird in Gegenwart von katalytisch wirksamen Mengen einer Base durchgeführt. Vorteilhaft verwendet man als Basen Alkali- oder Erdalkalihydroxide, Alkali- oder Erdalkalialkoholate z.B. von Alkoholen mit 1 bis 6 Kohlenstoffatomen. Besondere Bedeutung haben Natrium-, Kalium-, Lithium- oder Calcium-Alkoholate mit 1 - 4 Kohlenstoffatomen erlangt. Geeignet sind beispielsweise Natriummethylat oder Natriumethylat, Natrium- oder Kalium-tert.-butylat. Andere geeignete basischen Katalysatoren sind Alkali- oder Erdalkaliamide z.B. Natriumamid oder Lithiumamid. Besonders vorteilhaft sind geeignet Aminbasen, insbesondere tertiäre Amine, wie N-Dimethylanilin, Butyldimethylamin ferner Amidine wie 1,4-Diazabicyclo[2,2,2]octan oder 1,8-Diazabicyclo[5,4,0]undecen-7). Vorteilhaft setzt man Basen in einer Menge von 0,1 bis 30 mol-%, insbesondere 0,1 bis 10 mol-% bezogen auf Lactam ein.

Die Umsetzung wird bei einer Temperatur von 25 bis 300°C durchgeführt. Vorteilhaft wendet man eine Temperatur von 40 bis 100°C an.

Vorteilhaft setzt man je Mol Lactam 1,0 bis 5 mol Kohlensäure-Diester ein. Insbesonders hat es sich bewährt, je Mol Lactam 1,05 bis 4, vorzugsweise 1,2 bis 2,5 mol Kohlensäurediester anzuwenden, der hierbei gleichzeitig als Lösungsmittel dient.

In der Regel wird die Umsetzung bei Normaldruck durchgeführt. Es ist auch schwach erniedrigter oder schwach erhöhter Druck z.B. bis zu 2 bar anwendbar. Hierbei hält man zweckmäßig eine flüssige Phase ein. Die Umsetzung läßt sich diskontinuierlich und kontinuierlich durchführen.

Aus dem erhaltenen Reaktionsgemisch wird der Katalysator in der Regel mit Wasser ausgewaschen und anschließend ω-[O-substituierte Urethano]-carbonsäureester durch Destillation isoliert.

ω-(O-substituierte Urethano)-carbonsäurealkylester sind wertvolle Polymerbausteine oder eignen sich zur Herstellung von ω-Isocyanatocarbonsäureester.

Das Verfahren nach der Erfindung sei an folgenden Beispielen veranschaulicht.

### Beispiele

### Beispiel 1

In einem Rührkolben werden 508 g Caprolactam (4,5 mol), 1044 g Di-n-butylcarbonat (6 mol) und 12 g Natriummethylat (5 mol-%) bei 60°C gerührt. Nach einer Reaktionszeit von 2 h beträgt der Umsatz >98 %. Nach dem Waschen der organischen Phase mit Wasser und dem Abdestillieren von nicht umgesetztem Di-n-butylcarbonat isoliert man 1227 g 6-(O-Butylurethano)hexansäurebutylester (Ausbeute 95 %, Reinheit 96 %).

### Beispiel 2

113 g Caprolactam (1 mol) 94,5 g Dimethylcarbonat (1,05 mol) und 2,7 g Natriummethylat (5 mol-%) werden bei 130°C gerührt. Nach 0,5 h beträgt der Umsatz 98 %. Nach dem Waschen der organischen Phase mit Wasser und der destillativen Abtrennung von nicht umgesetztem Dimethylcarbonat isoliert man 189 g 6-(O-Methylurethano)hexansäuremethylester (Ausbeute 93 %).

### Beispiel 3

226 g Caprolactam (2 mol), 510 g Dimethallylcarbonat (3 mol) und 5,4 g Natriummethylat (0,5 mol-%) werden bei 60°C gerührt. Nach 1 h beträgt der Umsatz >98 %. Nach dem Waschen der organischen Phase mit Wasser erhält man nach der destillativen Abtrennung von nicht umgesetztem Dimethallylcarbonat 480 g 6-(O-Methallylurethano)hexansäuremethallylester (Ausbeute 85 %, Reinheit 97 %).

### Beispiel 4

113 g Caprolactam (1 mol), 180 g Dimethylcarbonat (2 mol) und 0,5 g Natriummethylat (1 mol-%) wurden bei 60°C gerührt. Nach einer Reaktionszeit von 2 h beträgt der Umsatz >97 %. Nach dem Waschen der organischen Phase mit Wasser und dem Abdestillieren von nicht umgesetztem Dimethylcarbonat isoliert man 193 g 6-(O-Methylurethano)-hexansäuremethylester (Ausbeute 95 %, Reinheit 96 %).

### Beispiel 5

113 g Caprolactam (1 mol), 180 g Dimethylcarbonat (2 mol) und 2 g Natriummethylat (5 mol-%) wurden bei 60°C gerührt. Nach einer Reaktionszeit von 4 h beträgt der Umsatz >95 %. Nach dem Waschen der organischen Phase mit Wasser und dem Abdestillieren von nicht umgesetztem Dimethylcarbonat und Caprolactam isoliert man 187 g 6-(O-Methylurethano)-hexansäuremethylester (Ausbeute 92 %, Reinheit 96 %).

### Beispiel 6

113 g Caprolactam (1 mol), 180 g Dimethylcarbonat (2 mol) und 2 g Natriumamid (5 mol-%) wurden bei 60°C gerührt. Nach einer Reaktionszeit von 10 h beträgt der Umsatz >90 %. Nach dem Waschen der organischen Phase mit Wasser und dem Abdestillieren von nicht umgesetztem Dimethylcarbonat isoliert man 173 g 6-(O-Methylurethano)-hexansäuremethylester (Ausbeute 85 %).

### Beispiel 7

21 g Butyrolactam (2-Pyrrolidinon) (0,25 mol), 29 g Dimethylcarbonat (0,32 mol) und 0,7 g Natriummethylat (5 mol-%) wurden bei 80°C zur Reaktion gebracht. Nach 8 h beträgt der Umsatz ca. 70 %. Nach dem Waschen der organischen Phase mit Wasser und dem Abdestillieren von nicht umgesetztem Dimethylcarbonat und Butyrolactam erhält man 4-(O-Methylurethano)-butansäuremethylester mit einer Selektivität von >95 %.

### Beispiel 8

25 g Valerolactam (2-Piperidinon) (0,25 mol), 29 g Dimethylcarbonat (0,32 mol) und 0,7 g Natriummethylat (5 mol-%) wurden bei 80°C umgesetzt. Nach 8 h beträgt der Umsatz ca. 80 %. Nach dem Waschen der organischen Phase mit Wasser und dem Abdestillieren von nicht umgesetztem Dimethylcarbonat und Valerolactam erhält man 5-(O-Methylurethano)-pentansäuremethylester mit einer Selektivität von >96 %.

### Beispiel 9

57 g Caprolactam (0,5 mol), 45 g Dimethylcarbonat (0,5 mol) und 10 g N,N-Dimethylanilin (16 mol-%) wurden im Autoklav bei 250°C zur Reaktion gebracht. Nach einer Reaktionszeit von 10 h beträgt der Umsatz 72 %. Nach dem Auswaschen der organischen Phase mit Wasser und Destillation erhält man 31 g 6-(O-Methylurethano)-hexansäuremethylester (Ausbeute 35 %).

## Patentansprüche

1. Verfahren zur Herstellung von ω-(O-substituierten Urethano)alkylcarbonsäureestern, dadurch gekennzeichnet, daß man ein Lactam mit 4 bis 9 Ringgliedern, das Alkyl-, Alkenyl-, Cycloalkyl- oder Aralkylreste mit 1 bis zu 12 Kohlenstoffatomen als Substitutenten haben kann, mit Kohlensäurediestern von Alkanolen, Alkenolen, Cycloalkanolen oder Aralkanolen mit bis zu 16 Kohlenstoffatomen bei einer Temperatur von 25 bis 300°C in Gegenwart von katalytisch wirksamen Mengen einer Base umsetzt.

2. Verfahren nach Anspruch 1, dadurch gekennzeichnet, daß man eine Temperatur von 40 bis 100°C einhält.

3. Verfahren nach den Ansprüchen 1 und 2, dadurch gekennzeichnet, daß man 0,1 - 30 mol-% katalytisch wirksame Base bezogen auf Lactam einsetzt.

4. Verfahren nach den Ansprüchen 1 bis 3, dadurch gekennzeichnet, daß man Alkalialkoholate von Alkanolen mit 1 bis 4 Kohlenstoffatomen, Alkali- oder Erdalkaliamide oder Amine als Basen verwendet.

5. Verfahren nach den Ansprüchen 1 bis 4, dadurch gekennzeichnet, daß man je Mol Lactam 1 bis 5 Mol Kohlensäurediester anwendet.

6. Verfahren nach den Ansprüchen 1 bis 5, dadurch gekennzeichnet, daß man Lactame mit 5 bis 9 Ringgliedern einsetzt.

7. Verfahren nach den Ansprüchen 1 bis 6, dadurch gekennzeichnet, daß man Kohlensäurediester von Alkanolen und Alkenolen mit 1 bis 4 Kohlenstoffatomen einsetzt.

## Claims

1. A process for preparing ω-(O-substituted urethano) alkylcarboxylic acid esters, which comprises reacting a lactam having 4 to 9 ring members which can have alkyl, alkenyl, cycloalkyl or aralkyl radicals having 1 to 12 carbon atoms as substituents, with carbonic acid diesters of alkanols, alkenols, cycloalkanols or aralkanols having up to 16 carbon atoms at from 25 to 300°C in the presence of catalytically active amounts of a base.

2. A process as claimed in claim 1, wherein a temperature of from 40 to 100°C is maintained.

3. A process as claimed in claims 1 and 2, wherein 0.1 - 30 mol % of catalytically active base is employed, based on lactam.

4. A process as claimed in claims 1 to 3, wherein alkali metal alkoxides of alkanols having 1 to 4 carbon atoms, alkali metal or alkaline earth metal amides, or amines are used as bases.

5. A process as claimed in claims 1 to 4, wherein from 1 to 5 mol of carbonic acid diester are used per mole of lactam.

6. A process as claimed in claims 1 to 5, wherein lactams having from 5 to 9 ring members are employed.

7. A process as claimed in claims 1 to 6, wherein carbonic acid diesters of alkanols and alkenols having 1 to 4 carbon atoms are employed.

## Revendications

1. Procédé de préparation d'esters d'acides ω-(uréthanno O-substitué)alkylcarboxyliques, caractérisé en ce que l'on fait réagir un lactame comportant 4 à 9 maillons cycliques, qui peut comporter, à titre de substituants, des radicaux alkyle, alcényle, cycloalkyle ou aralkyle, possédant de 1 à 12 atomes de carbone, avec des diesters de l'acide carbonique d'alcanols, d'alcénols, de cycloalcanols ou d'aralcanols, qui comportent jusqu'à 16 atomes de carbone, à une température de 25 à 300°C et en présence de proportions catalytiquement actives d'une base.

2. Procédé suivant la revendication 1, caractérisé en ce que l'on maintient une température de 40 à 100°C.

3. Procédé suivant les revendications 1 et 2, caractérisé en ce que l'on utilise de 0,1 à 30% molaires de base catalytiquement active par rapport au lactame.

4. Procédé suivant les revendications 1 à 3, caractérisé en ce que l'on utilise des alcoolates d'alcalis d'alcanols qui comportent de 1 à 4 atomes de carbone, des amidures de métaux alcalins ou de métaux alcalino-terreux ou des amines, à titre de bases.

5. Procédé suivant les revendications 1 à 4, caractérisé en ce que l'on utilise 1 à 5 moles de diester de l'acide carbonique par mole de lactame.

6. Procédé suivant les revendications 1 à 5, caractérisé en ce que l'on utilise des lactames qui comportent de 5 à 9 maillons cycliques.

7. Procédé suivant les revendications 1 à 6, caractérisé en ce que l'on utilise des diesters de l'acide carbonique d'alcanols et d'alcénols, qui comportent de 1 à 4 atomes de carbone.
